# EUROPEAN PATENT SPECIFICATION

(11) **EP 1 477 181 B1**
(45) Date of publication and mention of the grant of the patent: **06.12.2006**
(21) Application number: 03744706.7
(22) Date of filing: 22.01.2003
(51) Int. Cl.: A61K 38/08, C07K 7/06, A23K 1/165, A61P 33/00

(54) **METHOD OF STIMULATING GROWTH AND RESISTANCE TO DISEASES OF AQUATIC ORGANISMS**
VERFAHREN ZUR STIMULIERUNG DES WACHSTUMS UND DES WIDERSTANDS GEGEN ERKRANKUNGEN VON WASSERORGANISMEN
PROCEDE DE STIMULATION DE LA CROISSANCE ET DE LA RESISTANCE AUX MALADIES D'ORGANISMES AQUATIQUES

(30) Priority: 24.01.2002 CU 2002020
(43) Date of publication of application: 17.11.2004
(73) Proprietor: CENTRO DE INGENIERIA GENETICA Y BIOTECNOLOGIA, Ciudad Habana 12100 (CU)
(72) Inventor: ESTRADA GARCIA, Mario, Pablo, Ave.31 e/158 y 190, 12100 Ciudad de la Habana (CU); MARTINEZ RODRIGUEZ, Rebeca, Ave.31 e/158 y 190, 10600 Ciudad de la Habana (CU); ARENAL CRUZ, Amilcar, Circunvalacion norte, 70100 Camaguey (CU); PIMENTEL PEREZ, Rafael, Marcos, Circunvalacion n., 70100 Camaguey (CU); MORALES ROJAS, Antonio, Ave.31 e/158 y 190, 10600 Ciudad de la Habana (CU); PIMENTEL VAZQUEZ, Eulogio, Circunvalacion norte, 70100 Camaguey (CU); ESPINOSA VAZQUEZ, Alexander, 70100 Camaguey (CU); GARCIA MOLINA, Carmen, Aday, Circunvalacion norte, 70100 Camaguey (CU); CABRERA GONZALEZ, Edenaida, Circunvalacion norte, 70100 Camaguey (CU); VINJOY CAMPA, Mirta, C. Alegria e/ Ata. y Georgia, 10900 Ciudad de la Habana (CU); TOLEDO PEREZ, Sergio, 12000 Ciudad de la Habana (CU); CARRILLO FARNES, Olimpia, 11400 Ciudad de la Habana (CU); ZALDIVAR MUNOZ, Claudina, 10600 Ciudad de la Habana (CU)
(74) Representative: Winckels, Johannes Hubertus F.
(86) International application number: PCT/CU2003/000002
(87) International publication number: WO 2003/080102

(56) References cited:
- ES-A- 2 005 224
- US-A- 4 411 890
- US-A- 5 767 124

### SEQUENCE LIST

<110> CENTER FOR GENETIC ENGINEERING AND BIOTECHNOLOGY
<120> METHOD FOR THE GROWTHING STIMULATION AND RESISTANCE TO DISEASES OF AQUATIC ORGANISMS.
<130> aquatic organisms
<140>
   <141>
<150> CU 2002-0020
   <151> 2002-01-24
<160> 1
<170> PatentIn ver. 2.1
<210> 1
   <211> 6
   <212> PRT
   <213> artificial sequence
<220>
   <221> PEPTIDE
   <222> (1) .. (6)
   <223> GHRP6
<220>
   <223> Description of the Artificial sequence: GHRP-6
<400> 1

His D-Trp Ala Trp D-Phe Lys
1                    5

## Description

### Technical section

The invention relates to the application of chemical synthesis to increase the growth efficiency of fish and crustaceans of commercial value. The synthesis of analogous peptides of the Leu-encephaline and Met-encephaline type has been reported and its capacity to stimulate the release of the growth hormone in mammals and birds has been proved (Bowers C, Momany G, Reynolds G and A. Hong. 1984. Endocrinology. 114: 1537-45).

### Prior art

Studies to understand the structure-activity relation of growth hormone releasing peptides have been carried out in mammals and birds, identifying that the hexamer GHRP-6 (His-D-Trp-Ala-Trp-D-Phe-Lys-NH₂) is extremely powerful and safe to cause the release of the growth hormone, including in man (Bowers C, Momany G, Reynolds G and A. Hong, 1984. Endocrinology. 114: 1537-45), but there are no previous reports on the use of this compound in aquatic organisms.

Taking into account that there is no evidence in crustaceans to date that there is a cascade of signals occurring in growth stimulation (hypothalamus-hypophysis-white organ), it is shown for the first time that the peptide GHRP-6 on its own is capable of exerting a similar biological function in crustaceans.

Aquaculture is dominated by the production of freshwater fish; however, over the past decade the contribution of marine algae, molluscs and crustaceans to the global production of aquaculture has increased. A better understanding of the genetics, reproduction, nutrition and physiology of the organism selected is the first step in contributing to its productive development (Gómez-Chiarri M, Smith GJ, de la Fuente J and Powers DA. 1998. In de la Fuente J and Castro FO, editors. Gene transfer in aquatic organisms. Austin, Texas: RG Landes Company and Germany: Springer Verlag; p. 107-125).

Knowing the molecular characteristics of the hormones influencing the growth of marine species of economic importance can be used to achieve a better productive behaviour of these species in cultivation.

As an example of what has been said earlier can be mentioned the use of the gonadotropin releasing hormone (GnRH) and an antagonist of the dopamine receptor used by Silverstein and colleagues in 1999 (Silverstein JT., Bosworth BG., and Wolters WR. 1999. Journal of the World Aquaculture Society, vol. 30, no. 2, June, 263-268) to regulate and induce reproduction in catfish (*Ictalarus punctatus*), a species of great economic importance in world aquaculture.

Another interesting result of the use of synthetic peptides to increase productivity in farm animals was reported by Hashizune and colleagues in 1997 (Hashizume T., Sasaki M., Tauchi S. and Masuda H. 1997. Animal Science and Technology. Vol. 68, no. 3, March, 247-256) when they proved the induction of the growth hormone in goats by the application of injections with a novel synthetic growth hormone releasing peptide.

The supply of hormones such as insulin has also been tested in fish in oral form, proving that it produces changes in signals of receptors and hormones occurring in the adaptation of carp to different temperatures (Vera MI., Romero F., Figueroa J., Amthauer R., Leon G., Villanueva and Krauskopf M. 1993. Comp. Biochem. Physiol. Vol. 106A, no. 4, 677-682).

Other synthetic variants of growth hormone releasing peptide have also been studied in mammals, such as reported in 1995 by Patchett and colleagues with the peptide MK-0677, designed as a powerful oral activator of the growth hormone in dogs, without causing effects in the levels of tyrosine and prolactin after its application (Patchett AA., Nargund RP., Tata JR., Chen MH., Barakat KJ., Johnston DBR., Cheng K., Chan WWS., Butler B., Hickey G., Jacks T., Schleim K., Pong SS., Chaung LYP., Chen HY., Frazier E., Leung KH., Chiu SHL. and Smith RG. 1995. Proc. Natl. Acad. Sci. USA. Vol. 92, 7001-7005).

Growth hormone releasing hormones have also been tested in cows in order to increase milk production since increasing the circulating levels of GH stimulates milk production amongst other things (Soliman EB., Hashizume T., Ohashi S. and Kanematsu S. 1997. Domestic animal endocrinology. Vol. 14(1), 39-46).

In fish there are no previous reports on the use of the peptide GHRP-6 so its use to stimulate growth, increase survival and increase resistance to pathogenic agents reported in this document constitute a major solution for promoting and increasing the productivity of the cultivation of aquatic organisms.

Although advances have been made in the domestication and genetic crossing of some species of shrimps such as *Penaeus japonicus* and *Litopenaeus vannamei,* there has not been sufficient progress due to the lack of understanding of the genetic and biochemical processes of these species (Benzie, J.A.H., 1998. Penaeid genetics and biotechnology. Aquaculture 164, 23-47; Fjalestadl, K.T., Carr, W.H., Lotz, J.L., Sweeney, J.N., 1999. Aquaculture 173, 10), so one of the applications of major importance in penaeid shrimps could be genetic engineering focussed on improving growth and increasing their resistance to pathogenic agents (Bachere, E., Mialhe, E., Noel, D., Boulo, V., Morvan, A., Rodriguez, J. (1995) *Aquaculture* 132, 17-32).

This invention relates to the fact that the peptide GHRP-6 is able on its own to stimulate growth, improve the quality of the larvae, increase defences against pathogenic agents and increase the dry weight, concentration of proteins and RNA in the muscle of fish, crustaceans and molluscs.

### Advantages of the solution proposed

The present invention presents for the first time the use of the peptide GHRP-6 for stimulating growth and resistance to pathogenic agents and other diseases. It is also proved that the secretagog GHRP-6 on its own stimulates growth in fish and crustaceans by means of injection, by oral route or by immersion. Its effect in defending against pathogenic agents, increasing the dry weight in the muscle of the aquatic organisms treated, increasing the concentration of proteins and RNA in the tissue and maintaining erythrocytes is also demonstrated as proof of its harmlessness in tilapia.

### Brief description of the figures

Figure 1. Levels of GH in serum and of messenger RNA of IGF-1 in the liver of juvenile tilapias injected with the secretagog GHRP-6. Each group of three animals was injected intraperitoneally. Samples of serum and liver were taken 15, 30, 60 and 360 minutes after injection. Samples of serum were taken from each animal prior to injection. * significant difference over the control group (p < 0.001 ANOVA, Duncan test). tiGH: Tilapia growth hormone. The bars indicate the standard error.
Figure 2. Net and specific growth of the tilapias treated intraperitoneally with the peptide GHRP-6 and a control group over three weeks. * t-test p = 0.029. The bars indicate the variation in weight ± the standard error (SE) and the triangles the specific growth rate of both groups.
Figure 3. Net and specific growth of the tilapias treated orally with the peptide GHRP-6 in aqueous solution and its control group. The duration of the experiment was three weeks. * t-test p = 0.015. The bars indicate the variation in weight (± SE) and the triangles the specific growth rate of both groups (± SE).

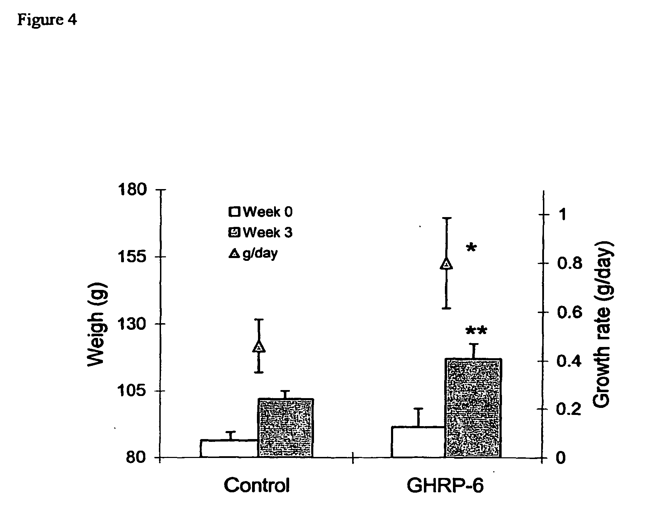

Figure 4. Net and specific growth of the tilapias treated orally with encapsulated peptide GHRP-6 and its control group. The duration of the experiment was three weeks. * t-test p = 0.05, ** t-test p = 0.007. The bars indicate the variation in weight (± SE) and the triangles the specific growth rate of both groups (± SE).

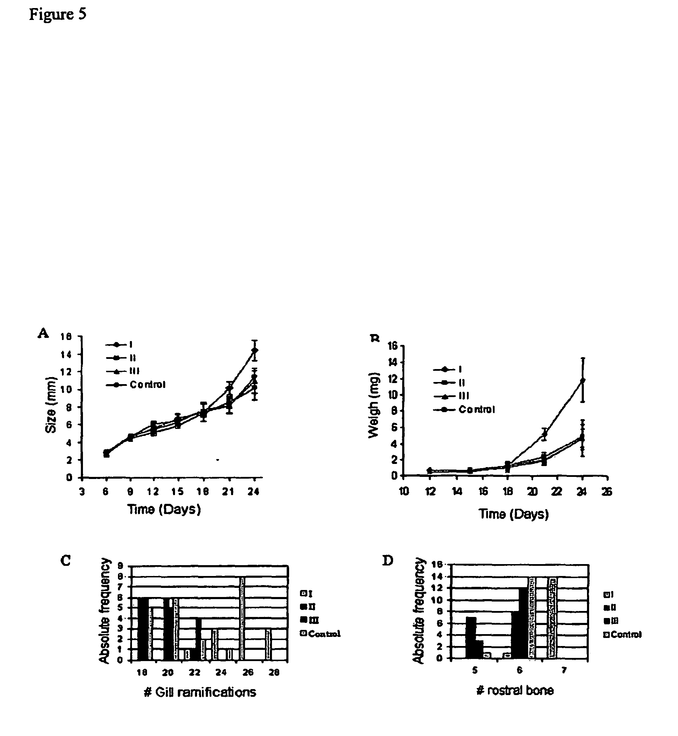

Figure 5. Growth experiment in shrimps Litopenaeus schmitti treated with the peptide GHRP-6. A: Profile of the weight increase in milligrams of the shrimps in the three groups treated with the peptide and the control group. B: Profile of the length increase in millimetres of the shrimps in the three groups treated with the peptide and the control group. C: Distribution of the absolute frequency of the branchial branches of three groups treated with the peptide and the control group. D: Distribution of the absolute frequency of the rostral modifications of three groups treated with the peptide and the control group.
   I, II, III: Groups treated with different concentrations of peptide.
   Control group treated with BSA.
Figure 6. Profile of the dry weight in animals treated with the peptide and controls. *** t-test p < 0.001. The bars indicate the variation in weight (± SD).
   *** p < 0.001. The bars indicate the variation in weight (± SD). ANOVA followed by a DUNCAN in the case of size and weight. Kolmogorov-Smmirnov in the branchial branches and rostral modifications.
Figure 7. Relations between RNA, protein and DNA. As can be seen in the figure, there are significant differences which indicates hypertrophy of these parameters in the animals treated with peptide. *** t-test p < 0.001. The bars indicate the variation in weight (± SD).
   *** p < 0.001. The bars indicate the variation in weight (± SD). ANOVA followed by a DUNCAN in the case of size and weight. Kolmogorov-Smmirnov in the branchial branches and rostral modifications.
Figure 8. Net growth under production conditions for shrimps treated with immersion baths with the peptide GHRP-6 in their larval stage and the control group with BSA. The duration of the experiment was six weeks. *** t-test p < 0.001. The bars indicate the variation in weight (± SD).
   *** p < 0.001. The bars indicate the variation in weight (± SD). ANOVA followed by a DUNCAN in the case of size and weight. Kolmogorov-Smmirnov in the branchial branches and rostral modifications.

### Detailed description of the invention

To prove growth stimulation in aquatic organisms, more specifically in fish, crustaceans and molluscs, using only the hexapeptide GHRP-6 "growth hormone releasing peptide" with the sequence: His-D-Trp-Ala-Trp-D-Phe-Lys-NH₂, it was first proved that in fish it was possible to effect a biological activity similar to that shown in mammals and birds (Bowers C, Momany G; Reynolds G and A. Hong. 1984. Endocrinology. 114: 1537-45).

For this, injections of the peptide were administered to juvenile tilapias and samples of serum and liver of the same were obtained at different times, proving how, as in mammals and birds, after injection of the peptide the circulating levels of the growth hormone in serum increase 15 minutes and half an hour after an increase in the messenger RNA of IGF-1 in liver was observed (Example 1).

Taking into account the fact that there is no evidence to date that the cascade of signals occurring in growth stimulation, hypothalamus-hypophysis-white organ (liver), is maintained exactly the same in aquatic organisms as in mammals and birds, it is shown for the first time that the peptide GHRP-6 on its own and administered by different routes is able to exert its activity in fish.

Experiments comparing growth in juvenile tilapias were carried out, administering the GHRP-6 to an experimental group and saline solution was administered to the control group, in each case depending on the administration route. Three administration routes were used: a) intraperitoneal injection; b) oral; c) immersion.

In the three cases, a significant rise was observed in the increase in the daily weight of the tilapias treated with the peptide GHRP-6 compared with a control group in each case (Example 2).

Another very valuable experiment for proving the effect of the peptide GHRP-6 in fish was to prove its applicability in tilapias of 1 mg weight by the immersion route and study the effect that this produced in the defence against pathogens and in the quality of their muscle (Example 3).

The results show that the group of tilapias treated with the peptide increased significantly in weight, showed a lower intensity index and extent of invasion of pathogenic agents than the control group and exhibited a lower water content in their muscle together with a greater concentration of proteins which suggests that the increase in weight produced by the peptide causes an increase in the protein synthesis and not an increase in the water content in their muscle.

The secretagog GHRP-6 was also tested in shrimps *Litopenaeus schmitti,* proving that it was able on its own to stimulate growth in crustaceans from its application by three different administration routes: a) intramuscular injection; b) oral; c) immersion (Example 4).

The secretagog GHRP-6 was tested in shrimps *Litopenaeus schmitti,* proving that it was able to stimulate growth in shrimps from its application of 4 immersion baths at different larval stages. At the end of the cultivation cycle of the larvae in a spawning centre, the groups treated with similar doses to tilapia exhibited greater quality. This was evidenced in an increase in weight, increase in size, a greater number of pairs of branchial branches and rostral modifications, a lower water content in the muscle of the animals and greater concentration of RNA protein in the muscle which was evidenced by a greater metabolic activity.

The larvae treated with the peptide and the control were introduced into earth ponds to see their productive behaviour. On the day of harvesting of the ponds, the animals treated with the peptide maintained the increase in weight, size and a greater homogeneity in weight and size in this group compared with the control.

### (Example 5)

The secretagog GHRP-6 was tested in shrimps *Litopenaeus schmitti,* proving that it is able to stimulate growth in shrimps by means of intramuscular injection in adult shrimps. At the end of the 15 days of the experiment, the peptide injected stimulated growth of the individuals between 100-150% compared with the control. With significant differences (p < 0.001).

### (Example 6 and 6.1)

The secretagog GHRP-6 was tested in shrimps *Litopenaeus schmitti,* proving that it is able to stimulate growth in shrimps when administered orally in shrimp larvae. The peptide GHRP-6 included in the diet increased the growth of the shrimps between 30-40% compared with the control. With very significant differences (p < 0.001). The secretagog GHRP-6 bioencapsulated in *Artemia salina* increased the growth of the individuals between 30 and 40% compared with the control. With highly significant differences (p < 0.001).

### EMBODIMENTS

### Example 1. Demonstration of the biological activity of GHRP-6 in fish

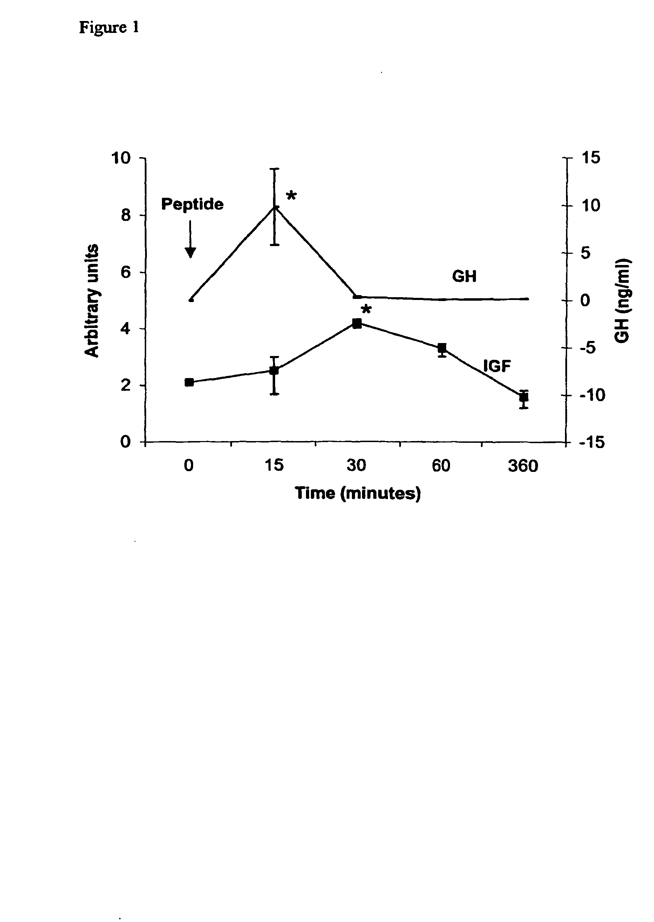

The levels of messenger RNA of IGF-1 in the liver of tilapias injected intraperitoneally with GHRP-6 were determined, and the GH levels in the same animals were monitored in the time, proving that the GHRP-6 was able in fish to stimulate the presence of the GH circulating in blood and how it responds to this in increasing the levels of messenger RNA of IGF-1 half an hour after injection of the peptide (Figure 1).

Fifteen tilapias with an average weight of 71 ± 28 g were used for the experiment. The peptide GHRP-6 was injected at a concentration of 0.1 µg/gbw (micrograms of peptide per gram weight of the animal). The samples of liver and blood were collected prior to treatment and 15, 30, 60 and 360 minutes after injection of the peptide (three animals were used each time). The serum and blood were frozen and kept at -70°C until they were used for the ELISA to quantify the circulating GH or for extracting the messenger RNA in the case of the liver for quantification by southern blot of the IGF-1 levels.

The total RNA of the liver of the tilapias used in the experiment was purified according to the protocol described by Chomczynski and Sacchi. 20 µg total RNA were applied and run in a 1% agarose-formaldehyde gel, and finally they were transferred to a nylon membrane (Hybond N, Amersham UK) and hybridised with a marked probe corresponding to the complementary DNA of the tilapia IGF-1 (6) and subsequently rehybridised with a probe of the human gene of glyceraldehyde 3-phosphate dehydrogenase (GADPH, kindly donated by Dr Bryan Williams, Cleveland Foundation, OH, USA) for standardising the signals.

The hybridisation signals were quantified from digital processing of the radiography image using a Hewlett Packard Scanjet Plus scanner. The images were processed using the Bandleader computation program.

The GH levels in the serum of the tilapias injected were measured with an ELISA using two monoclonol antibodies against the tiGH (Muñoz et al, in preparation).

### Example 2. Experiment on growth in juvenile tilapias treated with GHRP-6

### 2.1 Growth acceleration in tilapias treated with GHRP-6 intraperitoneally (ip)

The peptide GHRP-6 (BACHEM, Switzerland) was diluted in a sodium phosphate buffer solution (PBS) and injected twice a week for three weeks at 0.1 µg peptide / g wet weight for each fish (gbw). The peptide was applied to a group of 8 male tilapias with an average weight of 61.41 ± 14.36 g and a control group of 7 male tilapias with an average weight of 61.58 ± 29.67 g which received PBS was used. The average weight was measured each week (Figure 1). All the animals in the experiment were labeled with a microchip (Stoelting Co. Wood Dale, USA).

### 2.2 Growth acceleration in tilapias treated with GHRP-6 orally

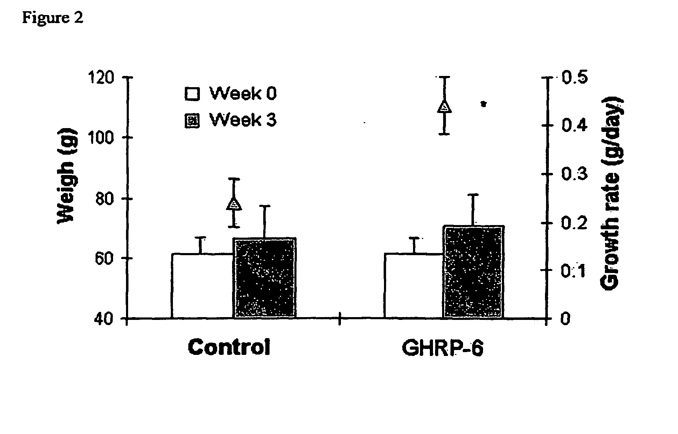

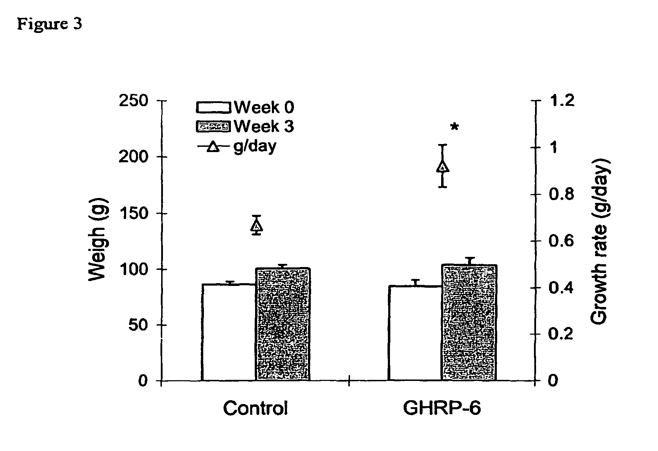

Two experiments were carried out for oral administration of the peptide GHRP-6. The first administering the peptide in liquid form dissolved in PBS and the second administering the peptide in the form of small beads encapsulated in calcium alginate according to the protocol previously published by Knorr and colleagues in 1988 (Figures 2 and 3 respectively). In both experiments, control groups to which saline solution was administered were used and in all cases tilapias intubated up to the pharyngeal cavity were used.

### 2.2.1 Peptide in aqueous solution

The peptide GHRP-6 (BACHEM, Switzerland) was diluted in PBS and administered orally by means of a plastic tube up to the pharyngeal cavity to a group of 7 male tilapias which had an average weight of 84.66 ± 12.2 g. A control group of 7 male tilapias with an average weight of 86.38 ± 6.26 g to which PBS was supplied under the same conditions as the group treated with the peptide, was taken. The treatment was carried out twice a week for three weeks, administering a dose of 0.1 µg peptide / g wet weight for each fish (gbw). The average weight was measured each week (Figure 2). All the animals in the experiment were labeled with a microchip (Stoelting Co. Wood Dale, USA).

### 2.2.2 Encapsulated peptide

Capsules were obtained for encapsulation of the peptide in accordance with the report by Knorr and colleagues in 1988. The encapsulated hexapeptide was administered through a plastic tube to the pharyngeal cavity to 7 tilapias with an average weight of 89.09 ± 8.38 g. Polymer beads without the peptide GHRP-6 were supplied by the same route to the control group consisting of 7 male tilapias with an average weight of 89.86 ± 13.54 g. The treatment was carried out twice a week for three weeks administering 0.1 µg/gbw on each occasion.

### Example 3. Growth stimulation in tilapias (Oreochromis sp) by means of the peptide GHRP-6 via immersion

Evaluation of growth in 1.5 g tilapia *Oreochromis sp.* was carried out using the peptide GHRP-6 in doses of 10 mg/100 ml and 100 mg/100 ml; the hematocryte value was also determined to have an idea of the state of the blood biochemistry in the animals treated with regard to the controls.

In this same experiment, the presence of trichodina and monogene helminths in the animals used in the test was studied to observe and compare the intensity and extent of the invasion of these pathogenic agents in the groups treated.

Finally, a study of the protein concentration and the percentage of moisture in the muscle of the tilapias studied was carried out.

Three groups with three replicas of 15 animals from a same spawning with an average initial weight of 1 g were selected for the test for which 9 x 40L ponds were used. All the animals were treated with the peptide by immersion once a week for 15 minutes for 45 days; the control group received the same treatment and immersion of them was carried out in saline solution.

The groups and treatments were distributed as follows:

| | |
|---|---|
| Group 1 | 10 µg/100 ml (treatment 1) |
| Group 2 | 100 µg/100 ml (treatment 2) |
| Group 3 | Negative control (saline solution) |

**Table 1. Study of weight. Immersion experiment with the peptide GHRP-6 in tilapias of 1 g weight over a period of 45 days.**

| Treatments | n | Average weight (g) ± SD | Comparison between groups | Difference |
|---|---|---|---|---|
| Group I | 25 | 4.56 ± 1.07 | I-II | 0.01454 |
| 10 µg/100 mL | | | | |
| Group II | 25 | 4.54 ± 1.38 | II-III | 1.07177* |
| 100 µg/100 mL | | | | |
| Group III | 25 | 3.47 ± 1.52 | III-I | 1.08632* |
| Saline solution | | | | |

| | | | | |
|---|---|---|---|---|
| ** Significant difference statistically. The multiple status test was applied to the samples.* | | | | |

**Table 2. Study of the hematocryte values. Immersion experiment with the peptide GHRP-6 in tilapias of 1 g weight over a period of 45 days.**

| Treatments | n | Average of the haematocryte ± SD | Comparison between groups | Statistical comparison |
|---|---|---|---|---|
| Group I | 15 | 27.46 ± 4.53 | I-II | (2.4)* |
| 10 µg/100 mL | | | | |
| Group II | 15 | 25.06 ± 5.25 | II-III | (1.0)* |
| 100 µg/100 mL | | | | |
| Group III | 15 | 26.46 ± 4.08 | III-I | (1.4)* |
| Saline solution | | | | |

| | | | | |
|---|---|---|---|---|
| ** There are no significant differences between the groups. The multiple status test was applied to the samples.* | | | | |

**Table 3. Results of the intensity of invasion (I) and extent of invasion (E) in the organisms treated for the cutaneous protozoon Trichodina sp. Immersion experiment with the peptide GHRP-6 in tilapias of 1 g weight over a period of 45 days.**

| Treatment | n | I^{a} | E (%)^{b} | Comparison between groups | Statistical comparison |
|---|---|---|---|---|---|
| Group I | 25 | 7.73 | 100 | I-II | (4.42) |
| 10 µg/100 mL | | | | | |
| Group II | 25 | 2.80 | 84.6 | II-III | (5.84)* |
| 100 µg/100 mL | | | | | |
| Group III | 25 | 8.76 | 92.30 | III-I | (1.42) |
| Saline solution | | | | | |

| | | | | | |
|---|---|---|---|---|---|
| ^{*a*}*I: intensity of invasion* ^{*b*}*E: extent of invasion* ** Significant difference statistically. The multiple status test was applied to the samples.* | | | | | |

**Table 4. Results of the intensity of invasion (I) and extent of invasion (E) in the organisms treated according to the values of the count of monogene helminths found in the gills of each fish. Immersion experiment with the peptide GHRP-6 in tilapias of 1 g weight over a period of 45 days.**

| Treatment | n | I^{a} | E (%)^{b} | Comparison between groups | Statistical comparison |
|---|---|---|---|---|---|
| Group I | 25 | 0.39 | 34.7 | I-II | (0.304) |
| 10 µg/100 mL | | | | | |
| Group II | 25 | 0.66 | 50 | II-III | (0.521) |
| 100 µg/100 mL | | | | | |
| Group III | 25 | 1.07 | 46 | III-I | (0.826)* |
| Saline solution | | | | | |

| | | | | | |
|---|---|---|---|---|---|
| ** Significant difference statistically. The multiple status test was applied to the samples.* | | | | | |

**Table 5. Percentage moisture values in the muscle tissue. Immersion experiment with the peptide GHRP-6 in tilapias of 1 g weight over a period of 45 days.**

| Treatment | n | Average humidity ± SD (%) | Comparison between groups | Statistical comparison |
|---|---|---|---|---|
| Group I | 24 | 82.96 ± 3.63 | I-II | (0.791) |
| 10 µg/100 mL | | | | |
| Group II | 24 | 83.5 ± 3.31 | II-III | (2.666)* |
| 100 µg/100 mL | | | | |
| Group III | 24 | 86.42 ± 3.23 | III-I | (3.458) |
| Saline solution | | | | |

| | | | | |
|---|---|---|---|---|
| ** Significant difference statistically. The multiple status test was applied to the samples.* *SD: standard deviation.* | | | | |

**Table 6. Values for the protein concentration in the muscle tissue. Immersion experiment with the peptide GHRP-6 in tilapias of 1 g weight over a period of 45 days**

| Treatment | n | Average protein concentration ±SD | Comparison between groups | Statistical comparison |
|---|---|---|---|---|
| Group I | 23 | 6.10 | I-II | (1.160)* |
| 10 µg/100 mL | | | | |
| Group II | 23 | 4.94 | II-III | (1.38)* |
| 100 µg/100 mL | | | | |
| Group III | 23 | 3.55 | III-I | (2.64)* |
| Saline solution | | | | |

| | | | | |
|---|---|---|---|---|
| ** Significant difference statistically. The multiple status test was applied to the samples* *SD: standard deviation.* | | | | |

### Example 4. Growth experiment in shrimps Litopenaeus schmitti by immersion baths with the peptide GHRP-6

Four groups of shrimp larvae were taken and four immersion baths were applied to them, one every three days for one hour's duration with different concentrations of the peptide GHRP-6 to three of them and one group was taken as control. The concentrations of the peptide used were: 0.001, 0.01 and 0.1 mg/L in groups I, II and III respectively; the control group was given the same frequency of immersion baths with BSA at 1 mg/L.

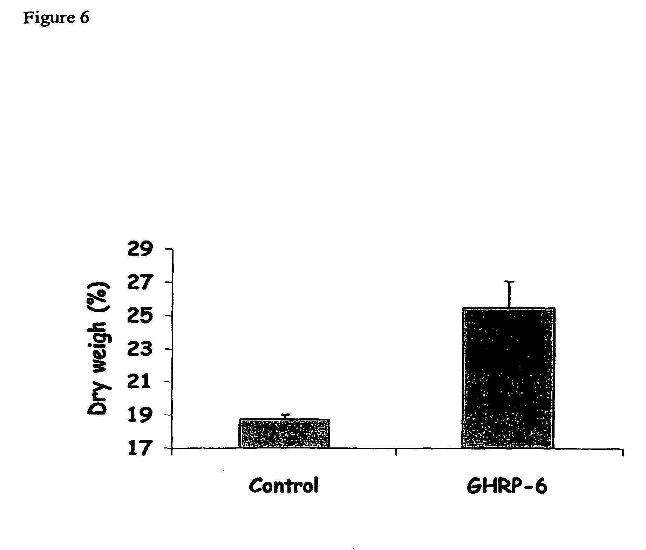

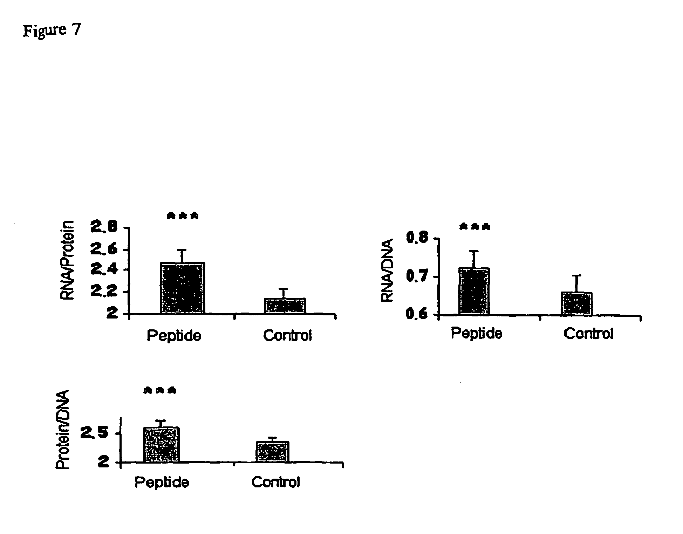

As a result it was observed that in the group treated with the greatest concentration of peptide 0.1 mg/L, the quality of the larvae of the shrimps *Litopenaeus schmitti* was improved. This was evidenced in an increase in weight of 125-153%, a 15-26% increase in size, a greater number of branchial branch pairs and rostral modifications. For each parameter the corresponding statistical test was carried out encountering in all cases highly significant differences (Figure 5). In addition it was found that the animals treated with GHRP-6 had a lower water content in the muscle and higher values for the RNA/DNA and protein/DNA ratios, giving an idea of what was activated in the muscle metabolism of these larvae (Figure 6 and 7).

These results are of great importance since when the growth of the shrimp is accelerated in the initial stages of its life, it causes a greater survival in the cultivation phase which is vital to increase the productivity of this crustacean of such commercial value in the world.

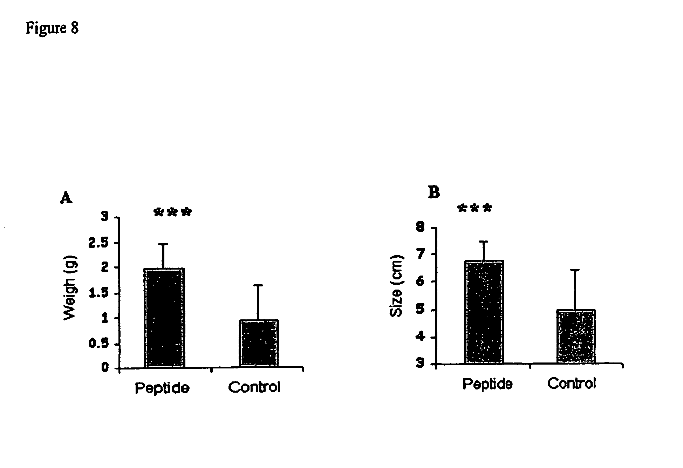

The previous idea was corroborated under production conditions in an increase in survival of 20% in the animals treated with GHRP-6 compared with their control brothers, also maintaining a 115% stimulation in weight and 37% in size, the animals treated with the peptide showing greater homogeneity in size than their untreated brothers, being reflected in the variation coefficient of just 24% and 9% in weight and size respectively in animals treated with the peptide versus 77% and 30% which is observed in the weight and size in the brother animals (Figure 8).

### Example 5. Growth stimulation in shrimps by means of intramuscular injection in animals of 15 g

Injections of 50 microL of peptide GHRP-6 were made between the first and second abdominal segment using an insulin syringe. The injections were carried out once every 3 days using 1 microg/g weight of the peptide GHRP-6. The control group was injected with 1 microg/g weight of BSA. 15 animals were used for each group. The effect of the peptide GHRP-6 was estimated by measuring the length of the shell with a slide gauge and weighed to a weight with 0.1 g error. The animals were placed in nylon bags (0.8 cm mesh) with dimensions 2 x 2 x 1 (length x width x height) in an earth pond with a salinity of 30g·L⁻¹, 25 C and natural photoperiod. The effect of the peptide GHRP-6 was estimated by measuring the length of the shell with a slide gauge and weighed to a weight with 0.1 g error.

The GHRP-6 injected at the different concentrations increased the growth of the individuals between 100-150% compared to the control. With significant differences (p < 0.001).

### Example 6. Growth stimulation in shrimps by inclusion in the diet

The peptide GHRP-6 was included in a diet for crustacean postlarvae at 1%. The peptide GHRP-6 included in the diet was microencapsulated by a complex coacervation method (Knorr D. and M. Daly. (1988). Process Biochemistry; 48-50). Subsequently *Litopenaeus schmitti* postlarvae were fed with the diet mentioned, and a control with BSA included in the feed. The tiGH effect was estimated by measuring the length of the shell of the larvae and postlarvae with an optical micrometer and weighed to a weight with 0.1 mg error.

The peptide GHRP-6 included in the diet increased the growth of the shrimps between 30-40% with regard to the control. With highly significant differences (p < 0.001).

### 6.1 Encapsulation in artemia

The peptide GHRP-6 was bioencapsulated in *Artemia* with which the *Litopenaeus schmitti* and *Litopenaeus vanamei* postlarvae were fed. To bioencapsulate the peptide GHRP-6 in the *Artemia salina,* it was added at a concentration of 10 mg/L for 1 hour and harvested and washed. The *Litopenaeus schmitti* postlarvae were then fed with this *Artemia* four times a day for one month experimentation. The control group was fed with *Artemia salina* with bioencapsulated BSA. The effect of the peptide GHRP-6 was estimated by measuring the length of the shell of the larvae and postlarvae with an optical micrometer and weighed to a weight with 0.01 mg error.

The peptide GHRP-6 bioencapsulated in *Artemia salina* increased the growth of the individuals between 30 and 40% with regard to the control. With highly significant differences (p < 0.001).

### Example 7. Growth experiment in salmon larvae (salmon salar) by immersion baths with peptide GHRP-6

Three groups of salmon larvae (salmon salar) were taken and six immersion baths were applied to them, one every three days for one hour duration with different concentrations of the peptide GHRP-6 to two of them and one group was taken as control. The concentrations of the peptide used were: 0.01 and 0.1 mg/L in groups I and II respectively; the same frequency of immersion baths with BSA at 1 mg/L was given to the control group.

As a result it was observed that in the group treated with the greater concentration of peptide 0.1 mg/L it increased in weight 120-145%, taking as reference 100% of the control group and an increase of 15-26% in size with regard to the control group was observed. On the other hand, it was found that the animals treated with GHRP-6 had a lower water content in the muscle, an increase in the values of the RNA/DNA and protein/DNA ratios, giving an idea of the activation of the metabolism in the muscle of these larvae.

These results are of great importance since when the growth of the salmon larvae in the larval stages is increased it causes greater survival in this phase of cultivation which is vital to increase the productivity of this species which is so important in the world's fish trade.

### Example 8. Effectiveness of the peptide GHRP-6 on the ectoparasite sea lice Caligus sp. in trout (Oncorhynchus mykiss)

Trout treated in their larval stages by means of immersion baths given every three days, with a total of 6 baths, with the peptide GHRP-6 at concentrations of 0.01 mg/L, 0.1 mg/L and a control group were placed in fattening cages in an area affected by the ectoparasite *Caligus sp.* From each cage were taken samples of five times 20 fish at intervals of one week. Both groups treated in their larval stages with the peptide GHRP-6 significantly reduced (p < 0.05) the content of adult chalimus and Caligus (two important types of sea lice) in relation to the control group, there being no difference between the groups treated.

## Claims

1. A method of increasing the productivity of fish or crustacean culture comprising feeding or administering to fish or crustaceans in culture the peptide His-D-Trp-Ala-Trp-D-Phe-Lys-NH₂ (GHRP-6) as sole active ingredient for stimulating their growth, in an amount effective to stimulate their growth.

2. A method according to claim 1, wherein said GHRP-6 peptide is given to the fish and crustaceans through formulated feed in a concentration of 0.01 - 50 µg of peptide per gram of wet weight of the animal.

3. A method according to claim 1, wherein said GHRP-6 peptide is given to the fish and crustaceans through periodic injections with intervals of from 1 to 7 days in a concentration of 0.05 - 10 µg of peptide per gram of wet weight of the animal.

4. A method according to claim 1, wherein said GHRP-6 peptide is given to the fish and crustaceans through immersion with intervals of from 1 to 7 days in fresh water or sea water having a concentration of 10 - 500 µg of peptide per liter.

5. A method according to claim 1, wherein said GHRP-6 peptide is given to the fish and crustaceans in encapsulated form through oral route with intervals of from 1 to 7 days in a concentration of 0.05 - 10 µg of peptide per gram of wet weight of the animal.

6. A method according to any one of claims 1-5, wherein said GHRP-6 peptide is given to tilapia *Oreochromis sp.*

7. A method according to any one of claims 1-5, wherein said GHRP-6 peptide is given to *Salmon sp..*

8. A method according to any one of claims 1-5, wherein said GHRP-6 peptide is given to shrimps *Litopenaeus schmitti, Litopenaeus vanamei.*

9. A method according to any one of claims 1-5, wherein said GHRP-6 peptide is given to shrimps *Penaeus sp.*

10. Use of the peptide His-D-Trp-Ala-Trp-D-Phe-Lys-NH₂ (GHRP-6) for preparing a composition for treatment of fish and crustaceans to increase their resistance to diseases.

11. Use of the peptide His-D-Trp-Ala-Trp-D-Phe-Lys-NH₂ (GHRP-6) for preparing a composition for preventive or therapeutic treatment of infection by parasites in fish and crustaceans.

12. Use according to claim 11, wherein said parasites comprise *Trichodina sp.* or *Helmintos monogeneos.*

13. Use according to claim 11, wherein said parasites comprise sea lice.

14. Use according to claim 11, wherein said parasites comprise ectoparasites.

## Patentansprüche

1. Verfahren zur Erhöhung der Produktivität von Fisch- oder Krebstierkultur, umfassend das Füttern oder Verabreichen des Peptids His-D-Trp-Ala-Trp-D-Phe-Lys-NH₂ (GHRP-6) an Fisch oder Krebstiere in Kultur als einzigen Wirkstoff zum Stimulieren ihres Wachstums, in einer Menge, die wirksam ist, ihr Wachstum zu stimulieren.

2. Verfahren gemäß Anspruch 1, wobei das GHRP-6-Peptid den Fischen und Krebstieren durch ein formuliertes Futter in einer Konzentration von 0,01- 50 µg Peptid pro Gramm Tier-Nassgewicht gegeben wird.

3. Verfahren gemäß Anspruch 1, wobei das GHRP-6-Peptid den Fischen und Krebstieren durch periodische Injektionen mit Abständen von 1 bis 7 Tagen in einer Konzentration von 0,05 - 10 µg Peptid pro Gramm Tier-Nassgewicht gegeben wird.

4. Verfahren gemäß Anspruch 1, wobei das GHRP-6-Peptid den Fischen und Krebstieren durch Eintauchen in Süß- oder Meerwasser, welches eine Konzentration von 10 - 500 µg Peptid pro Liter aufweist, mit Abständen von 1 bis 7 Tagen gegeben wird.

5. Verfahren gemäß Anspruch 1, wobei das GHRP-6-Peptid den Fischen und Krebstieren in gekapselter Form über den oralen Weg mit Abständen von 1 bis 7 Tagen in einer Konzentration von 0,05 - 10 µg Peptid pro Gramm Tier-Nassgewicht gegeben wird.

6. Verfahren gemäß einem der Ansprüche 1 - 5, wobei das GHRP-6-Peptid dem Buntbarsch *Oreochromis sp.* gegeben wird.

7. Verfahren gemäß einem der Ansprüche 1 - 5, wobei das GHRP-6-Peptid *Salmon sp*. gegeben wird.

8. Verfahren gemäß einem der Ansprüche 1 - 5, wobei das GHRP-6-Peptid den Garnelen *Litopenaeus schmittii, Litopenaeus vanamei* gegeben wird.

9. Verfahren gemäß einem der Ansprüche 1 - 5, wobei das GHRP-6-Peptid *Penaeus sp*. Garnelen gegeben wird.

10. Verwendung des Peptids His-D-Trp-Ala-Trp-D-Phe-Lys-NH₂ (GHRP-6) zum Herstellen einer Zusammensetzung für die Behandlung von Fisch und Krebstieren, um ihre Widerstandskraft gegenüber Erkrankungen zu erhöhen.

11. Verwendung des Peptids His-D-Trp-Ala-Trp-D-Phe-Lys-NH₂ (GHRP-6) zum Herstellen einer Zusammensetzung für die vorbeugende oder therapeutische Behandlung von Parasiteninfektion bei Fisch und Krebstieren.

12. Verwendung gemäß Anspruch 11, wobei die Parasiten *Trichodina sp.* oder *Helmintos monogeneos* umfassen.

13. Verwendung gemäß Anspruch 11, wobei die Parasiten Seeläuse umfassen.

14. Verwendung gemäß Anspruch 11, wobei die Parasiten Ektoparasiten umfassen.

## Revendications

1. Procédé pour augmenter la productivité d'une culture de poissons ou de crustacés, comprenant l'alimentation ou l'administration aux poissons ou aux crustacés en culture en peptide His-D-Trp-Ala-Trp-D-Phe-Lys-NH₂ (GHRP-6) en tant qu'unique ingrédient actif pour stimuler leur croissance, en une quantité efficace pour stimuler leur croissance.

2. Procédé selon la revendication 1, dans lequel ledit peptide GHRP-6 est fourni aux poissons et aux crustacés via des aliments formulés en une concentration de 0,01 à 50 µg de peptide par gramme de poids humide de l'animal.

3. Procédé selon la revendication 1, dans lequel ledit peptide GHRP-6 est fourni aux poissons et aux crustacés par des injections périodiques à des intervalles allant de 1 à 7 jours en une concentration de 0,05 à 10 µg de peptide par gramme de poids mouillé de l'animal.

4. Procédé selon la revendication 1, dans lequel ledit peptide GHRP-6 est fourni aux poissons et aux crustacés par immersion à des intervalles allant de 1 à 7 jours dans de l'eau douce ou de l'eau de mer ayant une concentration de 10 à 500 µg de peptide par litre.

5. Procédé selon la revendication 1, dans lequel ledit peptide GHRP-6 est fourni aux poissons et aux crustacés sous une forme encapsulée par voie orale à des intervalles allant de 1 à 7 jours en une concentration de 0,05 à 10 µg de peptide par gramme de poids mouillé de l'animal.

6. Procédé selon l'une quelconque des revendications 1 à 5, dans lequel ledit peptide GHRP-6 est fourni au tilapia *Oreochromis sp.*

7. Procédé selon l'une quelconque des revendications 1 à 5, dans lequel ledit peptide GHRP-6 est fourni à *Salmon sp.*

8. Procédé selon l'une quelconque des revendications 1 à 5, dans lequel ledit peptide GHRP-6 est fourni aux crevettes *Litopenaeus schmitti, Litopenaeus vanamei.*

9. Procédé selon l'une quelconque des revendications 1 à 5, dans lequel ledit peptide GHRP-6 est fourni aux crevettes *Penaeus sp.*

10. Utilisation du peptide His-D-Trp-Ala-Trp-D-Phe-Lys-NH₂ (GHRP-6) pour la préparation d'une composition destinée au traitement des poissons et des crustacés pour faire augmenter leur résistance aux maladies.

11. Utilisation du peptide His-D-Trp-Ala-Trp-D-Phe-Lys-NH₂ (GHRP-6) pour la préparation d'une composition destinée au traitement préventif ou thérapeutique d'une infection par des parasites chez les poissons et les crustacés.

12. Utilisation selon la revendication 11, dans laquelle lesdits parasites comprennent *Trichodina sp.* ou *Helmintos monogeneos.*

13. Utilisation selon la revendication 11, dans laquelle lesdits parasites comprennent les poux de mer.

14. Utilisation selon la revendication 11, dans laquelle lesdits parasites comprennent les ectoparasites.
